Europäisches Patentamt

European Patent Office

Office européen des brevets

(19)

(11) Veröffentlichungsnummer: **0 001 082**
**B1**

(12) **EUROPÄISCHE PATENTSCHRIFT**

(21) Anmeldenummer: **78100766.1**

(22) Anmeldetag: **28.08.78**

(51) Int. Cl³: **C 07 C 69/96,**
**C 07 C 68/06**

(54) **Verfahren zur Herstellung von Dialkylcarbonaten**

(30) Priorität: **07.09.77 DE 2740243**

(43) Veröffentlichungstag der Anmeldung:
**21.03.79 Patentblatt 79/06**

(45) Bekanntmachung des Hinweises auf die Patenterteilung:
**23.07.80 Patentblatt 80/15**

(84) Benannte Vertragsstaaten:
**BE CH DE FR GB LU NL**

(56) Entgegenhaltungen:
**US - A - 2 915 529**
**US - A - 3 803 201**

(73) Patentinhaber: **Bayer AG**
**Zentralbereich Patente, Marken und Lizenzen**
**D - 5090 Leverkusen 1, Bayerwerk (DE)**

(72) Erfinder: **Buysch, Hans-Josef, Dr.**
**Brandenburgerstrasse 28**
**D - 4150 Krefeld (DE)**
**Krimm, Heinrich, Dr.**
**Heyenbaumstrasse 65**
**D - 4150 Krefeld (DE)**
**Rudolph, Hans, Dr.**
**Haydnstrasse 9**
**D - 4150 Krefeld (DE)**

Courier Press, Leamington Spa, England.

Verfahren zur Herstellung von Dialkylcarbonaten

Die vorliegende Erfindung betrifft ein Verfahren zur Herstellung von Dialkylcarbonaten durch Umsetzung von Glykolcarbonaten mit Alkoholen in Gegenwart von Alkalimetallen und/oder Alkaliverbindungen.

Aus der US—PS 3 642 858 ist die Herstellung von Dialkylcarbonaten durch Umesterung von Alkylencarbonaten mit Alkoholen in Gegenwart von Alkalimetallen oder Alkaliverbindungen bekannt. Die Verwendung der Alkalimetalle oder Alkaliverbindungen als Katalysatoren erfolgt dabei in Mengen von 0,3 bis 0,01 Gew.—%, bezogen auf das Reaktionsgemisch. Als typische Reaktionstemperaturen werden solche zwischen 175 und 225°C angegeben. Niedrigere Temperaturen führen gemäß dem Beispiel 3 der Tabelle der US—PS auch bei längeren Reaktionszeiten zu geringen Umsätzen. Unter den Reaktionsbedingungen gemäß der US—PS finden zum Teil im beträchtlichen Maße Nebenreaktionen statt, die die Ausbeute vermindern und die Aufarbeitung erschweren. Insbesondere finden solche Nebenreaktionen bei den aliphatischen Carbonaten, die weniger stabil sind, statt. Diese spalten vor allem leicht Kohlendioxid ab unter Bildung von Äthern. Solche Nebenreaktionen finden bevorzugt zwischen Dialkylcarbonaten und 1,2-Glykolen statt, besonders leicht zwischen Glykolcarbonaten und 1,2-Glykolen, wenn im Verlauf der Umesterung der Glykolanteil zunimmt. Die sich bildenden Nebenprodukte sind vor allem Alkylglykoläther und Polyglykole wie Di-, Tri- und Tetraglykole.

Es wurde ein Verfahren zur Herstellung von Dialkylcarbonaten durch Umsetzung von Glykolcarbonaten mit Alkoholen bei erhöhter Temperatur in Gegenwart von Alkalimetallen oder Alkaliverbindungen gefunden, das dadurch gekennzeichnet ist, daß man bei der Umsetzung weniger als 0,01 Gew.—% an Alkalimetallen und/oder Alkaliverbindungen, bezogen auf das Reaktionsgemisch, einsetzt.

Bei der erfindungsgemäßen Umsetzung ist überraschend, daß mit den geringen Mengen an Alkalimetallen und/oder Alkaliverbindungen die Umesterung schneller verläuft als bei dem Verfahren der oben zitierten US—PS, und daß kaum $CO_2$-Abspaltung und Nebenreaktionen stattfinden.

Das erfindungsgemäße Verfahren besitzt gegenüber dem Stand der Technik folgende Vorteile: Die Katalysatormengen sind gering und betragen nur einen Bruchteil der üblicherweise verwendeten Menge. Der Katalysator kann daher ohne Schwierigkeit vom Reaktionsgemisch abgetrennt und wieder verwendet werden. Die durch hohe Katalysatormengen verursachten Zersetzungen des Reaktionsproduktes bei der destillativen Aufarbeitung des Reaktionsgemisches werden beim erfindungsgemäßen Verfahren vermieden. Die Reaktionstemperaturen können vergleichsweise niedrig gehalten werden, was Energiekosten einspart. Die Reaktionsgeschwindigkeit ist überraschenderweise trotz der geringen Katalysatormenge größer als bei dem in der US—PS beschriebenen Verfahren, was eine erhöhte Raum/Zeit-Ausbeute bedingt. Nebenreaktionen, wie die Polyglykolbildung, werden stark zurückgedrängt und dadurch Ausbeuteverluste vermieden.

Ausgangsprodukte für das erfindungsgemäße Verfahren sind einerseits aliphatische und/oder cycloaliphatische Hydroxyverbindungen mit 1 bis 10, bevorzugt mit 1 bis 6, besonders bervorzugt mit 1 bis 4 C-Atomen wie Methanol, Äthanol, Propanol, Isopropanol, n-Butanol, iso-Butanol, Allylalkohol, Amylalkohol, Cyclohexanol, Äthylcyclohexanol, Benzylalkohol und Methylglykol, bevorzugt Methanol und Äthanol, andererseits Carbonate von 1,2-Diolen mit 2 bis 4 Kohlenstoffatomen wie Äthylenglykolcarbonat, Propylenglykolcarbonat, Butylencarbonat, Vinyl-äthylenglykolcarbonat, Chlormethyl-äthylenglykolcarbonat, besonders bevorzugt sind Äthylenglykolcarbonat und Propylenglykolcarbonat.

Das Molverhältnis der Reaktionspartner ist nicht sehr entscheidend. Ein Überschuß an Alkohol von etwa 1 bis 10 Mol pro Mol Glykolcarbonat empfiehlt sich jedoch, um das Gleichgewicht in Richtung des gewünschten Carbonats zu verschieben. Natülich kann auch ein größerer Überschuß eingesetzt werden.

Geeignete Katalysatoren für das erfindungsgemäße Verfahren sind Alkalimetalle wie Lithium, Natrium, Kalium, Rubidium, Cäsium bevorzugt Lithium, Natrium, Kalium, und/oder Alkalimetallverbindungen wie die Hydride, Oxide, Hydroxide, Alkoholate und Amide sowie die Alkalimetallsalze von organischen Säuren wie Essigsäure, Propionsäure, Buttersäure, Benzoesäure, Stearinsäure, die Alkalimetallsalze der Kohlensäure wie Carbonate und Bicarbonate, die Alkalimetallsalze, die sich von anorganischen Säuren ableiten, wie der Salzsäure, Bromwasserstoff- und Jodwasserstoffsäure, der Salpetersäure, Schwefelsäure, Fluorwasserstoffsäure, Phosphorsäure, Blausäure, Cyanwasserstoffsäure und Rhodanwasserstoffsäure.

Die Mengen an Alkalimetallen und/oder Alkaliverbindungen liegen unter 0,01 Gew.—%. Im allgemeinen liegen sie in Bereich von 0,01 bis 0,0001 Gew.—%, vorzugsweise bei 0,009 bis 0,0005 Gew.—%, bezogen auf das Reaktionsgemisch.

Die Reaktionstemperatur beträgt etwa 50 bis 250°C, bevorzugt 100 bis 220°C, besonders bevorzugt 130 bis 200°C.

Die Reaktion kann drucklos durchgeführt werden. Bei niedrig siedenden Komponenten ist allerdings ein Druckreaktor erforderlich, wenn die Reaktion im oberen Temperaturbereich durchgeführt werden soll. Der Druck ist nicht kritisch. Im allgemeinen läßt man die Reaktion unter dem Eigendruck der Reaktanten ablaufen. Man kann allerdings auch unter erhöhtem Druck, z.B. unter einer Schutzgasatmosphäre, arbeiten. Dabei ist ein Druck von etwa 2 bis 100 bar zweckmäßig.

Die Produkte des erfindungsgemäßen Verfahrens sind geeignet als Lösungsmittel für Cellulosederivate und als Ausgangsprodukte für die Herstellung von Diarylcarbonaten, aliphatischen und aromatischen Polycarbonaten sowie für Arznei- und Pflanzenschutzmittel (vgl. DT—OS 2 528 412, DT—AS 1 031 512, J. Amer. Chem. Soc. 52, 314 (1930), Ullmanns Encyklopädie d. techn. Chemie, 3. Aufl., Bd. 9, S. 776 ff (1957)). Das erfindungsgemäße Verfahren sei anhand der nachfolgenden Beispiele näher erläutert, ohne es jedoch auf diese Beispiele einzuschränken.

### Beispiel 1

Ein Gemisch Von 672 g (21 Mol) Methanol, 370 g (4,2 Mol) Glykolcarbonat und 0,9 g Natriumchloracetat wird 2 Stunden bei 150°C gehalten. Nach Abkühlen wird aufdestilliert, wobei Methanol und Dimethylcarbonat zunächst bei Normaldruck, die höhersiedenden Komponenten im Vakuum entfernt werden. Um Substanzverluste zu vermeiden, werden 2 Kühlfallen der Destillation nachgeschaltet. Man erhält 730 g 31,4 Gew.—% Dimethylcarbonat enthaltendes Methanol, entsprechend 229 g Dimethylcarbonat. Das bedeutet, daß Glykolcarbonat zu 60,5 % in Dimethylcarbonat umgewandelt ist. Die höhersiedende Fraktion (61—82°/107 Pa; 290 g) besteht aus Glykol und nicht umgesetztem Glykolcarbonat, der Rückstand (1,0 g) aus Polyglykol. Die Aufarbeitungsverluste betragen ca. 2 Gew.—%.

### Vergleichsbeispiel

Dieses Beispiel stellt eine Nacharbeitung des Ansatzes 2 der Tabelle der US—PS 3 642 858 dar: Ein Gemisch von 640 g (13,9 Mol) Äthanol, 264 g (3,0 Mol) Glykolcarbonat und 3,0 g Natriumäthylat wird 3 Stunden auf 200°C erhitzt. Nach Abkühlen wird wie im Beispiel 1 aufgearbeitet. Man erhält eine Äthanolfraktion (687 g) mit 102 g Diäthylcarbonat entsprechend einem Umsatz an Glykolcarbonat zu Diäthylcarbonat von 29 %, eine Glykolfraktion (113 g 83—120°/400 Pa) und 59 g Rückstand an dickflüssigen Polyglykolen, aus denen die Glykolfraktion nur unter Zersetzungserscheinungen herausdestilliert werden kann. Der Aufarbeitungs- und Substanz- verlust (CO_2-Abspaltung) beträgt 5 Gew.—%. Ca. 30 % des eingesetzten Glykolcarbonates sind in Nebenprodukte übergegangen.

### Beispiel 2

Ein Gemisch von 640 g (13,9 Mol) Äthanol, 264 g (3,0 Mol) Glykolcarbonat und 0,08 g Natriumäthylat wird 3 Stunden auf 200°C erhitzt. Nach Aufarbeitung wie in Beispiel 1 erhält man eine Äthanolfraktion mit 170 g Diäthylcarbonat entsprechend einem Umsatz an Glykolcarbonat zu Diäthylcarbonat von 48 %, eine Glykolfraktion (220 g 62—90°; 67 Pa) mit ca. 57 % Glykolcarbonat und 2 g Polyglykol als Rückstand. Die Aufarbeitungsverluste betragen ca. 2 Gew.—%. Die Destillation verläuft ohne Zersetzung.

### Beispiel 3

Beispiel 1 wird wiederholt, wobei aber als Katalysator 0,05 g Natriumacetat eingesetzt werden. Die Aufarbeitung wie in Beispiel 1 ergibt: Der Umsatz an Glykolcarbonat zu Dimethylcarbonat beträgt 65 %, der Destillationsrückstand 0,2 g, d.h. Polyglykole sind praktisch nicht gebildet worden. Die Ausbeute an Dimethylcarbonat ist daher fast quantitativ, wenn Aufarbeitungsverluste von ca. 2 % berücksichtigt werden.

### Beispiel 4

Beispiel 1 wird wiederholt, allerdings mit 0,02 g Lithiumhydroxid als Katalysator. Man erhält einen Umsatz an Glykolcarbonat zu Dimethylcarbonat von 55 % und 0,3 g Rückstand.

### Beispiel 5

Beispiel 1 wird wiederholt mit 0,04 g Lithiumchlorid als Katalysator. Man erhält einen Umsatz an Glykolcarbonat von 47 % und 0,2 g Rückstand.

### Beispiel 6

Beispiel 1 wird wiederholt, aber statt 0,09 g Natriumchloracetat werden 0,02 g Natrium als Katalysator verwendet. Man erhält eine Umwandlung von Glykolcarbonat in Dimethylcarbonat von 62 % und einen Destillationsrückstand von 0,9 g.

### Beispiel 7

Beispiel 1 wird wiederholt mit einem Gemisch von 0,01 g Lithium und 0,03 g Natriumäthylat als Katalysator.

Man erhält eine Umwandlung von Glykolcarbonat in Dimethylcarbonat von 59 % und einen Destillationsrückstand von 1,1 g.

### Beispiel 8

Beispiel 1 wird wiederholt mit 0,003 g KOH als Katalysator.

Man erhält eine Umwandlung von Glykolcarbonat in Dimethylcarbonat von 29 % und einen Destillationsrückstand von 0,2 g.

### Beispiel 9

Ein Gemisch von 576 g (18 Mol) Methanol, 88 g (1 Mol) Glykolcarbonat und 0,025 g Natriummethylat wird unter Normaldruck zum Sieden erhitzt während über Kopf einer 1,7 m Füllkörperkolonne bei 63°C das Dimethylcarbonat/Methanol-Azeotrop abgenommen wird. Nach 45 Std. ist die Umesterung beendet und 290 g Destillat, entsprechend 85,5 g Dimethylcarbonat, sind abgetrennt.

Im Rückstand sind außer dem restlichen Methanol und dem gebildeten Glykol (59,8 g) keine höheren Glykole nachweisbar.

Ausbeute an Dimethylcarbonat: 95 % d.Th.

### Beispiel 10

Ein Gemisch von 672 g (21 Mol) Methanol, 459 g (4,5 Mol) Propylencarbonat und 0,07 g Natrium wird 2 h bei 200° gehalten. Nach Aufarbeitung wie in Beispiel 1 stellt man fest, daß 45% des eingesetzten Propylencarbonats in Dimethylcarbonat umgewandelt sind. Der Destillationsrückstand beträgt 0,7 g. Polyglykole sind also praktisch nicht entstanden.

### Vergleichsbeispiel:

Wiederholt man Versuch 10 mit 1 g Natrium statt 0,07 g, so erhält man eine 43 %-ige Umwandlung von Propylencarbonat in Dimethylcarbonat und der Destillationsrückstand beträgt 17 g an Polyglykolen.

### Patentansprüche

1. Verfahren zur Herstellung von Dialkylcarbonaten durch Umsetzung von Glykolcarbonaten mit Alkoholen bei erhöhter Temperatur in Gegenwart von Alkalimetallen oder Alkaliverbindungen, dadurch gekennzeichnet, daß man bei der Umsetzung weniger als 0,01 Gew.—% an Alkalimetallen und/oder Alkaliverbindungen, bezogen auf das Reaktionsgemisch, einsetzt.

2. Verfahren nach Anspruch 1, dadurch gekennzeichnet, daß man 0,009 bis 0,0005 Gew.—% an Alkalimetallen und/oder Alkaliverbindungen, bezogen auf das Reaktionsgemisch, einsetzt.

3. Verfahren nach Anspruch 1, dadurch gekennzeichnet, daß man bei Temperaturen im Bereich von 50 bis 250°C arbeitet.

4. Verfahren nach Anspruch 1, dadurch gekennzeichnet, daß man bei Temperaturen im Bereich von 100 bis 220°C arbeitet.

### Revendications

1. Procédé de préparation de carbonates de dialkyle par réaction de carbonates de glycols avec des alcools à chaud en présence de métaux alcalins ou de composés alcalins, ce procédé ce caractérisant en ce que dans la réaction, on met les métaux alcalins et/ou composés alcalins en oeuvre en quantité inférieure à 0,01% du poids de mélange de réaction.

2. Procédé selon la revendication 1, caractérisé en ce que l'on met en oeuvre les métaux alcalins et/ou composés alcalins en quantité de 0,009 à 0,0005% du poids du mélange de réaction.

3. Procédé selon la revendication 1, caractérisé en ce que l'on opère à des températures dans l'intervalle de 50 à 250°C.

4. Procédé selon la revendication 1, caractérisé en ce que l'on opère à des températures dans l'intervalle de 100 à 220°C.

### Claims

1. A process for the preparation of dialkyl carbonates by reacting glycol carbonates with alcohols at an elevated temperature in the presence of alkali metals or alkali metal compounds, characterised in that less than 0.01% by weight of alkali metals and/or alkali metal compounds, relative to the reaction mixture, are employed in the reaction.

2. A process according to claim 1, characterised in that 0.009 to 0.0005% by weight of alkali metals and/or alkali metal compounds, relative to the reaction mixture, are employed.

3. A process according to claim 1, characterised in that the reaction is carried out at temperatures in the range of 50° to 250°C.

4. A process according to claim 1, characterised in that the reaction is carried out at temperatures in the range of 100° to 220°C.